(19) Europäisches Patentamt
European Patent Office
Office européen des brevets

(11) **EP 4 270 991 A1**

(12) **EUROPEAN PATENT APPLICATION**
published in accordance with Art. 153(4) EPC

(43) Date of publication:
**01.11.2023 Bulletin 2023/44**

(21) Application number: **21910324.9**

(22) Date of filing: **08.12.2021**

(51) International Patent Classification (IPC):
*H04R 17/00* (2006.01)     *H04R 31/00* (2006.01)
*A61B 8/00* (2006.01)

(52) Cooperative Patent Classification (CPC):
**A61B 8/00; H04R 17/00; H04R 31/00**

(86) International application number:
**PCT/JP2021/045197**

(87) International publication number:
**WO 2022/138175 (30.06.2022 Gazette 2022/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.12.2020   JP 2020215569**

(71) Applicant: **Mitsubishi Pencil Company, Limited
Tokyo 140-8537 (JP)**

(72) Inventors:
• **YAMADA, Kunitaka
Fujioka-shi, Gunma 375-8501 (JP)**
• **OHNO, Toshiki
Fujioka-shi, Gunma 375-8501 (JP)**

(74) Representative: **Vossius & Partner
Patentanwälte Rechtsanwälte mbB
Siebertstraße 3
81675 München (DE)**

(54) **BACKING MATERIAL FOR ULTRASONIC PROBE, METHOD FOR MAKING SAME, AND ULTRASONIC PROBE**

(57)     A backing material for an ultrasonic probe according to the present invention comprises a carbonaceous matrix that has communicating holes distributed in the matrix, and a resin with which the communicating holes are filled.

EP 4 270 991 A1

**(Cont. next page)**

# FIG. 1

(a)

(b)

**Description**

FIELD

The present invention relates to a backing material for an ultrasonic probe, to a method for its production, and to an ultrasonic probe.

BACKGROUND

Ultrasonic diagnostic devices and ultrasonic imaging scanners for medical use send ultrasonic signals to the target location and receive the reflected signal from the target (echo signal), creating an image of the target. Such ultrasonic diagnostic devices and ultrasound imaging scanners mainly employ array-type ultrasonic probes having an ultrasonic signal transmission/reception function.

An ultrasonic probe is equipped with an acoustic lens, acoustic matching layer, piezoelectric element and backing material, in that order from the target side. The backing material of the ultrasonic probe must exhibit performance including satisfactory attenuation of ultrasonic waves to increase sensitivity, and high thermal conductivity to prevent overheating of the piezoelectric element. Various types of backing materials have been proposed.

PTL 1 discloses an ultrasonic probe having a piezoelectric element, an acoustic matching layer and an acoustic lens laminated in that order on an acoustic backing material sheet, the piezoelectric element and acoustic matching layer being divided into an array of sections and grooves being formed in the acoustic backing layer corresponding to the divided sections, wherein the acoustic backing material includes a vinyl ethylene-acetate copolymer with a vinyl acetate content of 20 to 80 mass% and a filler in the vinyl ethylene-acetate copolymer, and the acoustic impedance is 2 to 8 MRayls.

PTL 2 discloses an ultrasonic probe, wherein a backing member is provided on an ultrasonic transducer that transmits ultrasonic waves to an object, on the side opposite from the direction of transmission of the ultrasonic waves to the object, and wherein the backing member is constructed of a laminar backing material, a heat conductor comprising a material with higher thermal conductivity than the backing material, and a thermal conductive plate, the heat conductor being embedded in the backing material and formed in a columnar fashion reaching to both sheet surfaces of the backing material, and the thermal conductive plate being provided on at least the ultrasonic transducer side among both sheet surfaces of the backing material.

PTL 3 discloses a backing material for an ultrasonic probe that essentially consists of porous amorphous carbon.

PTL 4 discloses a method for producing a carbon porous body, wherein the method comprises loading particles of a chlorinated vinyl chloride resin into a container and firing them in an inert atmosphere.

[CITATION LIST]

[PATENT LITERATURE]

[PTL 1] JP 2006-33801
[PTL 2] JP 2013-115537
[PTL 3] JP 2019-193151
[PTL 4] JPS 59-64511

SUMMARY

[TECHNICAL PROBLEM]

The invention of PTL 3 can provide a backing material that is able to satisfactorily attenuate ultrasonic waves. However, the degree of attenuation is still in need of improvement. For an improved attenuation rate it is necessary to consider maintenance of thermal conductivity.

The present invention therefore provides a backing material having an improved attenuation rate for ultrasonic waves while maintaining thermal conductivity.

[SOLUTION TO PROBLEM]

After much ardent research, the present inventors have found that the problem can be solved by the following technical means, and the invention has thereupon been completed. Specifically, the present invention provides the following:

<Aspect 1> A backing material for an ultrasonic probe which has:

a carbonaceous matrix with communicating pores dispersed throughout the matrix, and
a resin filling at least a portion of the communicating pores.

<Aspect 2> The backing material according to aspect 1, wherein the acoustic impedance is 3.5 to 6.0 MRayl.
<Aspect 3> The backing material according to aspect 1 or 2, wherein the thermal conductivity according to JIS R1611-2010 is 7.0 W/m-K or greater.
<Aspect 4> The backing material according to any one of aspects 1 to 3, wherein the ultrasonic attenuation according to JIS Z 2354-2012 is -50 to -15 dB/cm.
<Aspect 5> The backing material according to any one of aspects 1 to 4, wherein the matrix further comprises a carbonaceous powder.
<Aspect 6> The backing material according to any one of aspects 1 to 5, wherein the density is 1.1 to 1.8 g/cm$^3$.
<Aspect 7> An ultrasonic probe equipped with an acoustic lens, an acoustic matching layer, a piezoelectric element and a backing material according to any one of aspects 1 to 6, in that order.
<Aspect 8> A method for producing a backing material for an ultrasonic probe, wherein the method includes:

providing a carbonaceous matrix with communicating pores dispersed throughout the matrix, and
filling a resin into the communicating pores of the matrix.

<Aspect 9> The method according to aspect 8, wherein provision of the matrix is by a method including the following steps:

mixing a curable resin, a dissipatable substance and a solvent to compatibility, to prepare a precursor composition, and
heat treating the precursor composition in a non-oxidizing atmosphere to carbonize the curable resin and form a matrix, and causing the dissipatable substance to disappear to form pores in the matrix.

[ADVANTAGEOUS EFFECTS OF INVENTION]

According to the invention it is possible to provide a backing material having an improved attenuation rate for ultrasonic waves while maintaining thermal conductivity.

BRIEF DESCRIPTION OF DRAWINGS

Fig. 1 is a conceptual drawing of a matrix forming a backing material for an ultrasonic probe of the invention. Fig. 1(a) shows a matrix of a first embodiment of the invention, and Fig. 1(b) shows a matrix of a second embodiment of the invention.

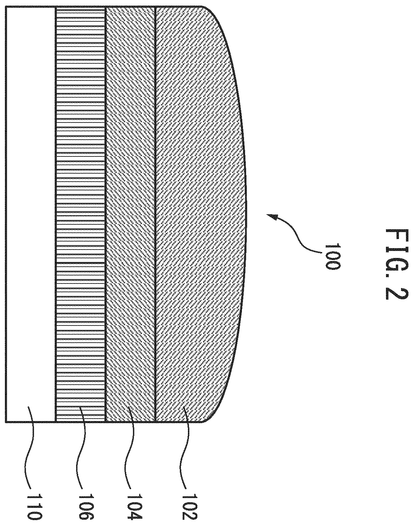

Fig. 2 is a conceptual drawing of an ultrasonic probe according to the invention.

DESCRIPTION OF EMBODIMENTS

«Backing material for ultrasonic probe»

The backing material for an ultrasonic probe of the invention has:

a carbonaceous matrix with communicating pores (communicating holes) dispersed throughout the matrix, and
a resin filling at least a portion of the communicating pores.

The backing material of the invention having such a construction is able to satisfactorily attenuate ultrasonic waves. More specifically, the ultrasonic attenuation of the backing material of the invention according to JIS Z 2354-2012 may be -15 dB/cm or lower, -17 dB/cm or lower or -18 dB/cm or lower, and -50 dB/cm or higher, -45 dB/cm or higher, -40 dB/cm or higher, -35 dB/cm or higher, -30 dB/cm or higher, -28 dB/cm or higher, -27 dB/cm or higher, -25 dB/cm or higher, -23 dB/cm or higher or -20 dB/cm or higher.

With no intention to be limited to any particular theory, it is thought possible that in the backing material of the invention, ultrasonic waves undergo diffuse reflection inside the matrix due to the porosity, thus resulting in the aforementioned ultrasonic attenuation. Since the backing material of the invention has the communicating pores of the matrix filled with a resin, it can exhibit high acoustic impedance and satisfactory ultrasonic attenuation and thermal conductive properties.

The thermal conductivity of the backing material of the invention, according to JIS R1611-**2010, may be 7.0 W/m·K or higher, 8.0 W/m·K or higher, 9.0 W/m·K or higher, 10.0 W/m·K or higher, 11.0 W/m-K or higher or 12.0 W/m·K or higher, and 300.0 W/m·K or lower, 250.0 W/m·K or lower, 200.0 W/m·K or lower, 150.0 W/m·K or lower, 100.0 W/m·K or lower, 70.0 W/m·K or lower, 50.0 W/m·K or lower, 25.0 W/m·K or lower, 15.0 W/m·K or lower, 14.0 W/m·K or lower, 13.5 W/m·K or lower, 13.0 W/m·K or lower, 12.0 W/m·K or lower, 11.0 W/m·K or lower or 10.5 W/m·K or lower.** The thermal conductivity can be measured in the thickness direction of the backing material using a Laser Flash Thermal Constants Analyzer (LFA457 by Netzsch Corp.).

The thickness of the backing material may be 5 mm or greater, 7 mm or greater, 10 mm or greater, 13 mm or greater, 15 mm or greater, 18 mm or greater or 20 mm or greater, and 100 mm or smaller, 90 mm or smaller, 80 mm or smaller, 70 mm or smaller, 60 mm or smaller, 50 mm or smaller, 40 mm or smaller, 35 mm or smaller, 30 mm or smaller or 25 mm or smaller.

The acoustic impedance of the backing material of the invention may be 3.5 MRayl or greater, 4.0 MRayl or greater or 4.5 MRayl or greater, and 6.0 MRayl or lower, 5.3 MRayl or lower, 5.0 MRayl or lower, 4.8 MRayl or lower, 4.5 MRayl or lower or 4.0 MRayl or lower.

The acoustic impedance is determined by the following formula.

$$\text{Acoustic impedance (Z: MRayl)} = \text{density } (\rho:\ g/cm^3) \times \text{acoustic velocity (C: m/sec)}/10^3$$

The acoustic velocity may be the acoustic velocity measured according to JIS Z 2353-2003, for example.

The density of the backing material may be 1.1 $g/cm^3$ or higher, 1.2 $g/cm^3$ or higher, 1.3 $g/cm^3$ or higher or 1.4 $g/cm^3$ or higher, and 1.8 $g/cm^3$ or lower, 1.7 $g/cm^3$ or lower, 1.6 $g/cm^3$ or lower or 1.5 $g/cm^3$ or lower. The density may be the density measured according to JIS Z 8807.

The bending strength of the backing material with this construction, according to JIS K 7074, may be 50 MPa or greater, 60 MPa or greater, 70 MPa or greater, 80 MPa or greater, 90 MPa or greater, 100 MPa or greater or 110 MPa or greater. The bending strength may also be 250 MPa or lower, 240 MPa or lower, 230 MPa or lower, 220 MPa or lower, 210 MPa or lower, 200 MPa or lower, 190 MPa or lower, 180 MPa or lower, 160 MPa or lower, 150 MPa or lower, 140 MPa or lower or 130 MPa or lower.

The bending strength is measured according to JIS K 7074. Specifically, a load (for 3-point bending) is applied to one point of a test piece simply supported on both ends , and the breaking load or maximum load for bending the test piece at a predetermined test rate is used for calculation by the following formula to determine the bending strength $\sigma_b$ (MPa).

$$\sigma_b = (3P_bL)/(2bh^2)$$

In the formula, L is the distance (mm) between points of support, b is the test piece width (mm), h is the test piece thickness (mm) and $P_b$ is the breaking load or maximum load (N). It should be noted that the test piece can be cut to any arbitrary size for the measurement.

The flexural modulus of the backing material with this construction, according to JIS K 7074, may be 10 GPa or greater, 11 GPa or greater, 12 GPa or greater, 13 GPa or greater, 14 GPa or greater, 15 GPa or greater or 16 GPa or greater. The flexural modulus may also be 35 GPa or lower, 33 GPa or lower, 30 GPa or lower, 29 GPa or lower, 28 GPa or lower, 26 GPa or lower, 24 GPa or lower, 22 GPa or lower or 20 GPa or lower.

The flexural modulus is measured according to JIS K 7074. Specifically, a load (for 3-point bending) is applied at one point of a test piece simply supported on both ends, and the load-deflection curve for bending the test piece at a predetermined test rate is recorded, using the initial slope of the linear portion of the load-deflection curve for calculation by the following formula to determine the flexural modulus $E_b$ (GPa).

$$E_b = (1/4) \times (L^3/bh^3) \times (P/\delta)$$

In the formula, L is the distance (mm) between points of support distance (mm), b is the test piece width (mm), h is the test piece thickness (mm) and P/$\delta$ is the slope (N/mm) of the linear portion of the load-deflection curve. It should be noted that the test piece can be cut to any arbitrary size for the measurement.

The constituent elements of the invention will now be described.

<Matrix>

The matrix is a carbonaceous matrix, with communicating pores dispersed throughout the matrix.

The term "carbonaceous" as used herein means composed of vitreous carbon and/or graphite.

The mean pore size of the pores forming the communicating pores of the matrix may be greater than 0 nm, 1 nm or greater, 3 nm or greater, 5 nm or greater, 8 nm or greater, 10 nm or greater, 15 nm or greater, 20 nm or greater, 30 nm or greater, 40 nm or greater, 50 nm or greater, 60 nm or greater, 70 nm or greater, 80 nm or greater or 90 nm or greater. The mean pore size of the pores may also be 100 $\mu$m or smaller, 90 $\mu$m or smaller, 80 $\mu$m or smaller, 70 $\mu$m or smaller, 65 $\mu$m or smaller or 60 $\mu$m or smaller. The mean pore size of the pores is the average diameter measured by the mercury intrusion method.

Measurement by the mercury intrusion method can be carried out under the following conditions using a measuring instrument such as an AutoPoreIV 9520 (Micromeritics Co.), with pretreatment of the matrix by drying at 100°C for 3 hours:

> Measurement range: 4 nm to 500 $\mu$m,
> Analysis: Washburn method,
> Surface tension: 480 dynes/cm,
> Contact angle: 140°.

The matrix may further comprise carbonaceous powder dispersed in the matrix.

Two embodiments of the matrix will be described below, but other types of porous matrices with communicating pores may be used for the backing material for an ultrasonic probe of the invention.

<Matrix: first embodiment>

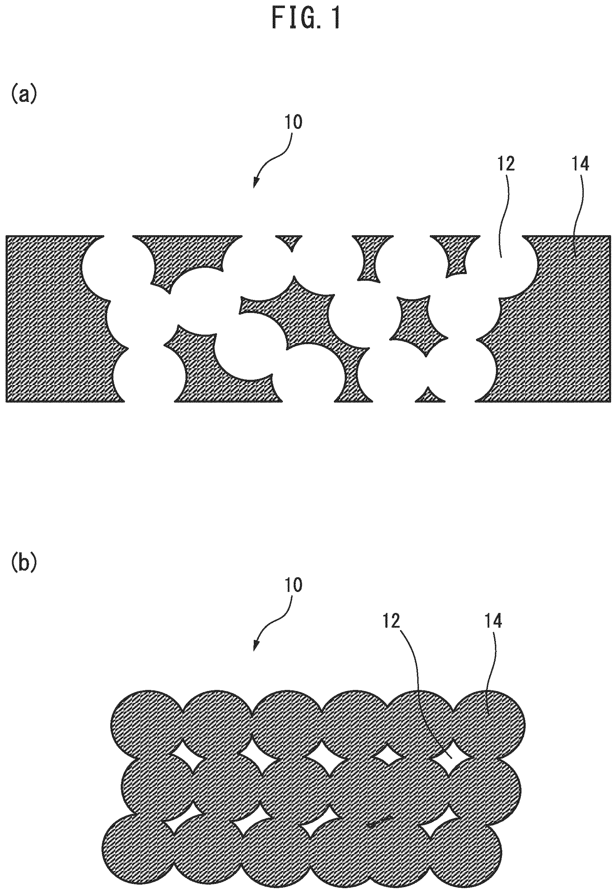

According to the first embodiment, as shown in Fig. 1(a), the matrix 10a has pores formed from a dissipatable pore-forming agent inside the vitreous carbon 14, the pores 12 may form the communicating pores.

The mean pore size of the pores forming the communicating pores of the matrix of the first embodiment may be greater than 0 nm, 1 nm or greater, 3 nm or greater, 5 nm or greater, 8 nm or greater, 10 nm or greater, 15 nm or greater, 20 nm or greater, 30 nm or greater, 40 nm or greater, 50 nm or greater, 60 nm or greater, 70 nm or greater, 80 nm or greater or 90 nm or greater. The mean pore size of the pores may also be 500 nm or smaller, 450 nm or smaller, 400 nm or smaller, 350 nm or smaller, 300 nm or smaller, 250 nm or smaller, 220 nm or smaller, 200 nm or smaller, 180 nm or smaller, 150 nm or smaller, 130 nm or smaller or 110 nm or smaller. The mean pore size of the pores is the average diameter measured by the mercury intrusion method, as explained above.

The thickness of the matrix of the first embodiment may be 5 mm or greater, 7 mm or greater, 10 mm or greater, 13 mm or greater, 15 mm or greater, 18 mm or greater or 20 mm or greater, and 100 mm or smaller, 90 mm or smaller, 80 mm or smaller, 70 mm or smaller, 60 mm or smaller, 50 mm or smaller, 40 mm or smaller, 35 mm or smaller, 30 mm or smaller or 25 mm or smaller.

<Matrix: second embodiment>

For the second embodiment, as shown in Fig. 1(b), the matrix 10b may have vitreous carbon particles 14 mutually connected, with pores 12 formed between the vitreous carbon particles and the pores 12 communicating with each other.

The mean pore size of the pores forming the communicating pores of the matrix of the second embodiment may be 1 $\mu$m or greater, 3 $\mu$m or greater or 5 $\mu$m or greater. The diameter of the pores may also be 100 $\mu$m or smaller, 90 $\mu$m or smaller, 80 $\mu$m or smaller, 70 $\mu$m or smaller, 65 $\mu$m or smaller or 60 $\mu$m or smaller. The mean pore size of the pores is the average diameter measured by the mercury intrusion method, as explained above.

The constituent elements of the matrix will now be described.

(Vitreous carbon)

The vitreous carbon can be obtained by carbonizing a precursor composition comprising a curable resin, a dissipatable substance and a solvent, for example. A specific method for producing the backing material is described below.

(Carbonaceous powder)

A carbonaceous powder may also be dispersed in the matrix.

Examples of carbonaceous powders include amorphous carbon powder, graphene, carbon nanotubes, graphite and carbon black. These may be used alone or in combinations.

The form of the carbonaceous powder is not particularly restricted, and examples includes flat, array and spherical shapes.

The mean particle size of the carbonaceous powder may be 10 nm or greater, 20 nm or greater, 30 nm or greater, 50 nm or greater, 70 nm or greater, 100 nm or greater, 200 nm or greater, 300 nm or greater, 500 nm or greater, 700 nm or greater, 1 $\mu$m or greater, 2 $\mu$m or greater or 3 $\mu$m or greater, and 150 $\mu$m or smaller, 140 $\mu$m or smaller, 130 $\mu$m or smaller, 120 $\mu$m or smaller, 110 $\mu$m or smaller, 100 $\mu$m or smaller, 90 $\mu$m or smaller, 80 $\mu$m or smaller, 70 $\mu$m or smaller, 60 $\mu$m or smaller, 50 $\mu$m or smaller, 40 $\mu$m or smaller, 30 $\mu$m or smaller, 20 $\mu$m or smaller, 18 $\mu$m or smaller, 15 $\mu$m or smaller, 13 $\mu$m or smaller, 10 $\mu$m or smaller or 7 $\mu$m or smaller. Throughout the present specification, the "mean particle size" is the median diameter (D50) calculated based on volume using laser diffraction.

The content of the carbonaceous powder in the matrix may be 80 mass% or lower, 75 mass% or lower, 70 mass% or lower, 65 mass% or lower, 60 mass% or lower, 55 mass% or lower, 50 mass% or lower, 45 mass% or lower, 40 mass% or lower, 35 mass% or lower, 30 mass% or lower, 25 mass% or lower, 20 mass% or lower or 15 mass% or lower, and 5 mass% or greater, 7 mass% or greater or 10 mass% or greater, based on the total mass of the matrix. A carbonaceous powder content of 50 mass% or lower will help facilitate shaping of the matrix. A carbonaceous powder content of 5 mass% or greater will also help ensure satisfactory mechanical properties for the matrix.

<Resin>

The resin is filled into the communicating pores. The resin may be selected in consideration of attenuation and workability, and examples include epoxy resins, urethane resins, phenol resins, rubber and elastomers, either alone or in combinations.

«Ultrasonic probe»

As shown in Fig. 1, the ultrasonic probe 100 of the invention is equipped with an acoustic lens 102, an acoustic matching layer 104, a piezoelectric element 106 and the backing material 108, in that order.

<Acoustic lens>

The acoustic lens is generally provided to focus the ultrasonic beam by refraction and improve the resolving power.

According to the invention, the material of the acoustic lens may be a conventionally known homopolymer such as silicone rubber, fluorine-silicone rubber, polyurethane rubber or epichlorohydrin rubber, or copolymer rubber such as ethylene-propylene copolymer rubber obtained by copolymerization of ethylene and propylene.

<Piezoelectric element>

The piezoelectric element is generally an element having an electrode and a piezoelectric material capable of sending and receiving ultrasonic waves to allow conversion of an electrical signal into mechanical oscillation or of mechanical oscillation into an electrical signal.

(Piezoelectric material)

The piezoelectric material may be a material that is able to convert an electrical signal into mechanical oscillation or mechanical oscillation into an electrical signal. Examples of piezoelectric materials include piezoelectric ceramics such as lead zirconate titanate (PZT)-based ceramics and PbTiOs-based ceramics, organic polymer piezoelectric materials such as vinylidene fluoride (VDF)-based polymers and vinylidene cyanide (VDCN)-based polymers, as well as crystals and Rochelle salt.

Examples of vinylidene fluoride (VDF)-based polymers include polyvinylidene fluoride (PVDF) and polyvinylidene fluoride-trifluoroethylene (P(VDF-TrFE)). Vinylidene cyanide (VDCN)-based polymers include polyvinylidene cyanide (PVDCN) and vinylidene cyanide-based copolymers.

(Electrode)

Examples of electrodes include gold (Au), platinum (Pt), silver (Ag), palladium (Pd), copper (Cu), aluminum (Al), nickel (Ni) and tin (Sn).

<Acoustic matching layer>

The acoustic matching layer is generally a layer that aligns acoustic impedance between the ultrasonic transducer and the subject, being composed of a material having acoustic impedance midway between the ultrasonic transducer and the subject.

Materials to be used in the acoustic matching layer include aluminum, aluminum alloys (such as Al-Mg alloy), magnesium alloy, Macor glass, glass, fused quartz, copper graphite, polyethylene (PE) or polypropylene (PP), polycarbonate (PC), ABC resin, polyphenylene ether (PPE), ABS resin, AAS resin, AES resin, nylon (PA6, PA6-6), PPO (polyphenylene oxide), PPS (polyphenylenesulfide: optionally containing glass fibers), PEEK (polyether ether ketone), PAI (polyamideimide), PETP (polyethylene terephthalate), , epoxy resins and urethane resins.

The acoustic matching layer may consist of a single layer or multiple layers.

<Method for producing backing material>

The method of the invention for producing the backing material includes:

providing a matrix made of vitreous carbon, with communicating pores dispersed throughout the matrix, and filling a resin into the communicating pores of the matrix.

<Provision of matrix: first embodiment>

For the first embodiment, the matrix can be provided by a method including the following steps:

compatibilizing a curable resin, a dissipatable substance and a solvent by mixing, to prepare a precursor composition, and

heat treating the precursor composition in a non-oxidizing atmosphere to carbonize the curable resin and form a matrix, and causing the dissipatable substance to disappear to form communicating pores in the matrix.

The method of the invention may further include shaving a backing material block obtained by carbonizing a precursor composition, to prepare the backing material.

When it is attempted to produce a matrix having a thickness of 5 mm or greater, a large amount of low-molecular-weight substances are generated at the curing stage and the initial carbonizing stage, thus greatly contracting the volume and causing interior accumulation of gas composed of the low-molecular-weight substances, and this has led to a problematic tendency toward cracking. The larger the thickness of the matrix to be produced, the more gas that is generated further (deeper) from the surface of the molded body and consequently the more volume contraction and gas generation takes place, resulting in greater stress on the carbon precursor during the carbonizing stage and tending to generate cracks.

The present inventors have found, however, that the method described above allows a matrix with a thickness of 5 mm or greater to be produced. Specifically, forming a compatible mixture of a curable resin, a dissipatable substance and a solvent can form independent paths throughout the carbon precursor for gas to escape from inside the carbon precursor at the carbonizing stage, thereby helping to satisfactorily reduce stress caused by gas accumulation, for production of the aforementioned vitreous carbon molded body without cracking.

(Fabrication of precursor composition)

The precursor composition is fabricated by mixing the curable resin, dissipatable substance and solvent to compatibilize them.

The mixing may be carried out by publicly known stirring means such as a Disper mixer.

(Molding of precursor composition)

The precursor composition may be molded by loading the precursor composition into a die and hardening it.

(Heat treatment of precursor composition)

Heat treatment of the precursor composition may include heat treating the precursor composition in a non-oxidizing atmosphere, carbonizing the curable resin to form the main body of the backing material, and causing the dissipatable substance to disappear to form pores in the backing material.

The heat treatment may be carried out by increasing the temperature to 800°C or higher, 850°C or higher or 900°C or higher, and 3000°C or lower, 2800°C or lower, 2500°C or lower, 2200°C or lower, 2000°C or lower, 1800°C or lower, 1600°C or lower, 1500°C or lower, 1400°C or lower, 1300°C or lower, 1200°C or lower, 1150°C or lower, 1100°C or lower, 1050°C or lower or 1000°C or lower, for example.

The substances used in the method for producing the backing material of the invention will now be described.

(Curable resin)

A curable resin is generally a resin that cures by three-dimensional crosslinking. Most preferably, the curable resin used is a curable resin that can be carbonized without thermal decomposition during heating to 1000°C in a non-oxidizing atmosphere, and that has a carbonization yield (residual carbonization yield) of 40% or greater.

Examples of curable resins include one or more curing precursors such as furan resins, phenol resins, epoxy resins, furan-phenol-based resins, phenol-modified furan co-condensation products, melamine resins, urea resins or furan-urea-based resins.

(Curing agent)

When the curable resin used is a furan resin, phenol-furan-based resin or furan-urea-based resin, for example, the curing agent may be an organic sulfonic acid-based curing agent such as para-toluenesulfonic acid.

(Dissipatable substance)

The dissipatable substance is a substance, especially an organic substance, that can disappear by thermal decomposition at a prescribed thermal decomposition temperature.

The thermal decomposition temperature can be determined by TG analysis in a nitrogen atmosphere at a temperature-elevating rate of 10°C/min. Specifically, the thermal decomposition temperature of the substance may be based on the weight reduction percentage W (%) at each measuring temperature T, and determined as the peak temperature for $dW/dT$ plotted by calculating $dW/dT$ at each temperature.

The thermal decomposition temperature of the dissipatable substance is preferably a lower temperature than the carbonizing temperature of the curable resin, and it is preferably 500°C or lower, 480°C or lower, 450°C or lower or 420°C or lower, for example. Such a thermal decomposition temperature will allow satisfactory formation of paths to allow escape of low-molecular-weight gas substances that are generated in the carbonization temperature range of the curable resin.

The thermal decomposition temperature is preferably 300°C or higher, 320°C or higher, 350°C or higher or 380°C or higher. A thermal decomposition temperature of 300°C or higher can drastically reduce contraction of the precursor composition caused by generation of large amounts of low-molecular-weight substances at the initial temperature in carbonizing, helping to inhibit closure of the aforementioned paths.

Examples of dissipatable substance s to be used include polyvinyl butyral (PVB), polyvinylpyrrolidone and polyethylene glycol.

When polyethylene glycol is used as the dissipatable substance, the molecular weight of the dissipatable substance is preferably 400 or higher, 600 or higher, 800 or higher, 1000 or higher, 3000 or higher, 5000 or higher, 8000 or higher, 10,000 or higher, 12,000 or higher, 14,000 or higher or 17,000 or higher, and 100,000 or lower, 90,000 or lower, 80,000 or lower, 70,000 or lower, 60,000 or lower, 50,000 or lower, 45,000 or lower, 40,000 or lower, 35,000 or lower, 30,000 or lower or 25,000 or lower, from the viewpoint of limiting the thermal decomposition temperature to within the range specified above. When dissipatable substance s with different molecular weights are used in admixture, the weighted average of the molecular weights weighted against the content of each component may be in the range specified above.

The dissipatable substance content is preferably greater than 0 mass%, 1 mass% or greater, 2 mass% or greater, 3 mass% or greater or 4 mass% or greater based on the solid mass of the precursor composition, from the viewpoint of satisfactorily forming the aforementioned paths, and preferably 10 mass% or lower, 9 mass% or lower, 8 mass% or lower, 7 mass% or lower, 6 mass% or lower or 5 mass% or lower, from the viewpoint of obtaining satisfactory mechanical strength for the matrix. The phrase "solid mass of the precursor composition" means the total mass of the curable resin and the dissipatable substance.

(Solvent)

The solvent of the invention is a solvent that can compatibilize the curable resin and the dissipatable substance. The term "compatibilize" as used herein means to bring to a state where undissolved substance can no longer be confirmed when the precursor composition before curing is observed under an optical microscope with at least 100x magnification.

The boiling point of the solvent is preferably 150°C or higher from the viewpoint of maintaining prolonged compatibility with the dissipatable substance and thus forming paths in a satisfactory manner. The boiling point may be 150°C or higher, 160°C or higher, 170°C or higher, 180°C or higher, 190°C or higher or 200°C or higher, and 300°C or lower, 280°C or lower or 250°C or lower.

Examples of solvents include alcohols such as benzyl alcohol, aprotic polar solvents such as N-methyl-2-pyrrolidone (NMP), dimethyl sulfoxide (DMSO), 1,3-dimethyl-2-imidazolidinone (DMI), N,N-dimethylformamide (DMF) and N,N-dimethyl acetamide (DMAC), glycol-based solvents such as propylene glycol, triethylene glycol, tetraethylene glycol and polyethylene glycol of molecular weight 600 or lower, and glycol ethers such as 3-methoxy-3-methyl-1-butanol (Solfit). Any one or more of these may also be used in admixture.

One example of a combination that may be used as the curable resin/dissipatable substance/solvent satisfying the solubility parameter conditions specified above is the following:

Furan resin/polyethylene glycol/benzyl alcohol + tetraethylene glycol, furan resin/polyethylene glycol/benzyl alcohol + triethylene glycol, furan resin/polyethylene glycol/benzyl alcohol + diethylene glycol, furan resin/polyvinylpyrrolidone/benzyl alcohol + tetraethylene glycol, phenol resin/polyethylene glycol + PVB/tetraethylene glycol + benzyl alcohol, furan resin/polyethylene glycol/NMP.

In this example, the combination can prepared in a homogeneous compatible state for use.

<Provision of matrix: second embodiment>

For the second embodiment, the matrix can be provided by a method including the following steps:

filling carbon precursor particles into a die and heat curing them to produce a molded composition, and
heat treating the molded composition in a non-oxidizing atmosphere to carbonize the carbon precursor particles.

The method of the second embodiment may be carried out with reference to PTL 4, for example.

(Fabrication of molded composition)

According to one aspect, the molded composition can be obtained by filling carbon precursor particles into a mold and heat curing them.

Examples for the carbon precursor particles include resin particles made of a polyvinyl chloride resin, phenol resin, furan resin, imide resin, epoxy resin or unsaturated polyester resin, as well as pitch such as coal tar pitch or petroleum pitch. These may be used alone, or two or more may be used in admixture.

According to a different embodiment, at least one of the resin particles and the pitch may be kneaded and extrusion molded, and the resulting molded body may be pulverized to obtain particles as the carbon precursor particles.

The mean particle size of the carbon precursor particles may be 1 $\mu$m or greater, 2 $\mu$m or greater, 3 $\mu$m or greater or 5 $\mu$m or greater, and 500 $\mu$m or smaller, 400 $\mu$m or smaller, 300 $\mu$m or smaller, 200 $\mu$m or smaller, 100 $\mu$m or smaller, 50 $\mu$m or smaller, 30 $\mu$m or smaller, 20 $\mu$m or smaller or 10 $\mu$m or smaller, for example. The mean particle size is the median diameter (D50) calculated based on volume using laser diffraction. Measurement by laser diffraction can be carried out using an MT3300II particle size distribution analyzer (Microtrac-Bell).

(Heat treatment)

Heat treatment may be carried out at a temperature of 600°C or higher, 650°C or higher, 700°C or higher, 750°C or higher, 800°C or higher, 850°C or higher or 900°C or higher, and 3000°C or lower, 2700°C or lower, 2500°C or lower, 2300°C or lower, 2100°C or lower, 2000°C or lower, 1700°C or lower, 1500°C or lower, 1300°C or lower, 1200°C or lower, 1150°C or lower, 1100°C or lower, 1050°C or lower or 1000°C or lower, for example.

<Filling of resin>

The resin may be filled by a publicly known method. The resin to be filled may be any of the ones mentioned above.

EXAMPLES

The present invention will now be explained in more specific detail through the following Examples and Comparative Examples, which are not intended to limit the invention in any way.

«Fabrication of backing material»

<Example 1>

A homogeneous solution was obtained by mixing 45 mass parts of a furan resin (VF303 by Hitachi Chemical Co., Ltd.) as a curable resin, 6 mass parts of polyethylene glycol with a molecular weight of 20,000 (thermal decomposition temperature: 426°C) as a dissipatable substance, 18 mass parts of benzyl alcohol (boiling point: 205°C) as a solvent and 13 mass parts of tetraethylene glycol (boiling point: 328°C), and thoroughly stirring with a Disper mixer. To the obtained solution there was added 18 mass parts of graphite (flaky graphite, Nippon Graphite, mean particle size: 5 $\mu$m), and the mixture was uniformly dispersed with a bead mill. To the dispersion there was added 1 mass part of para-toluenesulfonic acid as a curing agent, and the mixture was thoroughly stirred using a stirrer (MARK II Homomixer, type 2.5, Primix Corp.) and prepared by reduced-pressure defoaming.
The solution was cast into a frame with a 5 mm thickness and hardened, and then treated in argon gas at 2000°C to obtain a matrix with a thickness of 4 mm. The mean pore size of the obtained matrix was 0.1 $\mu$m.
For measurement of the mean pore size, the matrix was subjected to drying pretreatment at 100°C for 3 hours and then measured by mercury intrusion under the following conditions using an AutoPoreIV 9520 (Micromeritics).

Measurement range: about 4 nm to 500 $\mu$m
Analysis: Washburn method
Surface tension: 480 dynes/cm
Contact angle: 140°

The matrix was impregnated and filled with an epoxy resin and cured to obtain a backing material for Example 1. The physical properties were then evaluated.

<Comparative Example 1>

A backing material for Comparative Example 1 was obtained in the same manner as Example 1, but without impregnation and filling of an epoxy resin.

<Example 2>

After mixing 70 mass parts of polyvinyl chloride resin powder (M1008, Kaneka Corp.) and 30 mass parts of graphite (20 $\mu$m spherical graphite, Nippon Graphite) with an open mixer, the mixture was filled into a frame with a 5 mm thickness and heat cured. The mixture was treated in argon gas at 2000°C to obtain a matrix with a thickness of 4 mm. The mean pore size of the obtained matrix was 5 $\mu$m.
The matrix was impregnated and filled with an epoxy resin and cured to obtain a backing material for Example 2.

<Example 3>

After mixing 75 mass parts of polyvinyl chloride resin powder (M1008, Kaneka Corp.) and 25 mass parts of graphite (flaky graphite: 5 $\mu$m, Nippon Graphite) with an open mixer, an extrusion molding machine was used for molding into a linear form with a diameter of 2 mm. A pulverizer was used for pulverizing and the powder which passed through 36 mesh but failed to pass through 50 mesh was obtained. The powder was packed into a frame with a thickness of 5 mm and heat cured. The mixture was treated in argon gas at 2200°C to obtain a matrix with a thickness of 4 mm. The mean pore size of the obtained matrix was 55 $\mu$m.
The matrix was impregnated and filled with a urethane resin and cured, and then impregnated and filled with an epoxy resin and cured, to obtain a backing material for Example 3.

<Comparative Example 2>

To 70 parts of a furan resin (Hitachi Chemical Co., Ltd.) as an amorphous carbon source, 10 parts of graphite (flaky graphite, Nippon Graphite, mean particle size: 5 $\mu$m) and 20 parts of PMMA (Sekisui Plastics Co., Ltd., mean

particle size: 10 μm) as a pore-forming material there was added 1 part of *p*-toluenesulfonic acid as a curing agent, and the mixture was thoroughly stirred with a stirrer (MARK II Homomixer, type 2.5, Primix Corp.) and prepared by reduced-pressure defoaming. The solution was cast into a frame with a 5 mm thickness and hardened, and then treated in nitrogen gas at 1000°C to obtain a backing material with a thickness of 4 mm.

<Comparative Example 3>

An acrylic resin board (Misumi Co.) was used as the backing material for Comparative Example 3.

«Evaluation»

Each fabricated backing material was cut to a size of 30 mm × 30 mm × 3 mm and the mass was measured, calculating the density and conducting the following evaluation.

<Measurement of acoustic velocity and calculation of acoustic impedance>

The acoustic velocity inside each fabricated backing material was measured at 25°C using a sing-around sound velocity measuring device, according to JIS Z 2353-2003. The calculated density and measured acoustic velocity were used to calculate the acoustic impedance.

<Ultrasonic attenuation>

The ultrasonic attenuation inside each backing material was evaluated according to JIS Z 2354-2012, filling a water tank with water at 25°C, generating 1 MHz ultrasonic waves in the water using a JSR DPR500 Ultrasonic Pulser Receiver, and measuring the size of the amplitude before and after the ultrasonic waves passed through the backing material, to evaluate the ultrasonic attenuation.

<Thermal conductivity>

The thermal conductivity was measured in the thickness direction of the fabricated backing material using a Laser Flash Thermal Constants Analyzer (LFA457 by Netzsch Corp.).
The construction and evaluation results for the Examples and Comparative Examples are shown in Table 1.

[Table 1]

Table 1

| | Construction | | | | Evaluation | | | | |
| | Pore | | | Filling resin | Density (g/cm3) | Acoustic velocity (m/s) | Acoustic impedance (MRayl) | Ultrasonic attenuation (dB/cm) | Thermal conductivity (W/m·K) |
| | Presence of pores | Communication between pores | Pore size (μm) | | | | | | |
|---|---|---|---|---|---|---|---|---|---|
| Example 1 | + | + | 0.1 | Epoxy | 1.45 | 3500 | 5.1 | -24 | 10.0 |
| Comp. Example 1 | + | + | 0.1 | - | 0.9 | 3300 | 3.0 | -13 | 7.1 |
| Example 2 | + | + | 5 | | 1.48 | 2500 | 3.7 | -19 | 7.0 |
| Example 3 | + | + | 55 | Urethane + epoxy | 1.46 | 3000 | 4.4 | -26 | 13.0 |
| Comp. Example 2 | + | - | Not measurable | Not measurable | 1.10 | 3700 | 4.1 | -18 | 4.7 |
| Comp. Example 3 | - | - | - | Not measurable | 1.18 | 2700 | 3.2 | -15 | 0.2 |

From Table 1 it is seen that the backing materials of Examples 1 to 3, which had communicating pores and a resin filling the communicating pores, were backing materials with satisfactory ultrasonic wave attenuation and satisfactory heat conductivity.

The following Reference Examples are examples of vitreous carbon molded bodies forming the backing material of Example 1, i.e. molded bodies comprising porous vitreous carbon with communicating pores, for reference. While molding was with a thickness of 4 mm for convenience of evaluation in Example 1, the following Reference Examples demonstrate that it is possible to mold the backing material to a larger thickness with the construction of Example 1.

<Reference Example 1>

A homogeneous solution was obtained by mixing 120 mass parts of a furan resin (VF303 by Hitachi Chemical Co., Ltd.) as a curable resin, 14 mass parts of polyethylene glycol (PEG) with a molecular weight of 11,000 (thermal decomposition temperature: 426°C) as a dissipatable substance, 26 mass parts of benzyl alcohol (BA) (boiling point: 205°C) as a solvent and 40 mass parts of tetraethylene glycol (TEG) (boiling point: 328°C), and thoroughly stirring with a Disper mixer. The dissipatable substance content was 10 mass% based on the solid mass of the precursor composition.

To the obtained solution there was added 2 mass parts of para-toluenesulfonic acid (PTS) as a curing agent, and the mixture was stirred to homogeneity and subjected to reduced-pressure defoaming treatment to obtain a precursor composition. The precursor composition was filled into a die with a diameter of 100 mm and a thickness of 25 mm, and cured. The cured precursor composition was removed from the die and heat treated to a temperature of 1000°C under a nitrogen gas atmosphere to obtain a vitreous carbon molded body with a diameter of 80 mm and a thickness of 20 mm.

The obtained vitreous carbon molded body had a pore diameter of about 50 nm as measured by image analysis using SEM, a bending strength of 80 MPa, a flexural modulus of 19 GPa and an acoustic impedance of 4.5 MRayl.

<Reference Example 2>

A homogeneous solution was obtained by mixing 126 mass parts of a furan resin (VF303 by Hitachi Chemical Co., Ltd.) as a curable resin, 10 mass parts of polyethylene glycol with a molecular weight of 20,000 (thermal decomposition temperature: 426°C) as a thermally decomposable organic substance, 20 mass parts of Solfit (boiling point: 174°C) as a solvent and 30 mass parts of triethylene glycol (TrEG) (boiling point: 287°C), and thoroughly stirring with a Disper mixer. The dissipatable substance content was 7 mass% based on the solid mass of the precursor composition.

To the obtained solution there was added 14 mass parts of graphite (flaky graphite, Nippon Graphite, mean particle size: 5 $\mu$m), and the mixture was uniformly dispersed with a bead mill. To the obtained dispersion there was added 1 part by mass of para-toluenesulfonic acid as a curing agent, and the mixture was stirred to homogeneity and subjected to reduced-pressure defoaming treatment to obtain a precursor composition. The precursor composition was filled into a die with a diameter of 100 mm and a thickness of 30 mm, and cured. The cured precursor composition was removed from the die and heat treated to a temperature of 1400°C under a nitrogen gas atmosphere to obtain a vitreous carbon molded body with a diameter of 80 mm and a thickness of 25 mm.

The obtained vitreous carbon molded body had a pore diameter of about 50 nm as measured by image analysis using SEM, a bending strength of 96 MPa, a flexural modulus of 17.5 GPa and an acoustic impedance of 4.4 MRayl.

<Reference Example 3>

A homogeneous solution was obtained by mixing 80 mass parts of a furan resin (VF303 by Hitachi Chemical Co., Ltd.) as a curable resin, 2 mass parts of polyethylene glycol with a molecular weight of 20,000 (thermal decomposition temperature: 426°C) and 2 mass parts of polyethylene glycol with a molecular weight of 600 (thermal decomposition temperature: 390°C) as thermally decomposable organic substances, 10 mass parts of benzyl alcohol (boiling point: 205°C) and 10 mass parts of diethylene glycol (DEG) (boiling point: 244°C) as solvents, and thoroughly stirring with a Disper mixer.

To the obtained solution there was added 80 mass parts of amorphous carbon powder (mean particle size: 10 $\mu$m), and the mixture was uniformly dispersed with a bead mill or Disper mixer. To the obtained dispersion there was added 3 mass parts of para-toluenesulfonic acid as a curing agent, and the mixture was stirred to homogeneity and subjected to reduced-pressure defoaming treatment to obtain a precursor composition. The precursor composition was filled into a die with a diameter of 100 mm and a thickness of 30 mm, and cured. The cured precursor composition was removed from the die and heat treated to a temperature of 1000°C under a nitrogen gas atmosphere to obtain a vitreous carbon molded body with a diameter of 80 mm and a thickness of 25 mm.

The obtained vitreous carbon molded body had a pore diameter of about 50 nm as measured by image analysis using SEM, a bending strength of 115 MPa, a flexural modulus of 24 GPa and an acoustic impedance of 5.3 MRayl.

<Reference Comparative Example 1>

A homogeneous solution was obtained by mixing 120 mass parts of a furan resin (VF303 by Hitachi Chemical Co., Ltd.) as a curable resin, and 26 mass parts of benzyl alcohol (boiling point: 205°C) and 40 mass parts of tetraethylene glycol (boiling point: 328°C) as solvents, and thoroughly stirring with a Disper mixer.

To the obtained solution there was added 1 part by mass of para-toluenesulfonic acid (PTS) as a curing agent, and the mixture was stirred to homogeneity and subjected to reduced-pressure defoaming treatment to obtain a precursor composition. The precursor composition was filled into a die with a diameter of 100 mm and a thickness of 30 mm, and cured. When the cured precursor composition was removed from the die and heat treated to a temperature of 1400°C under a nitrogen gas atmosphere, large cracks and minute internal cracks were found to be present, and a vitreous carbon molded body could not be obtained. Consequently it was not possible to measure the pore size, bending strength, flexural modulus and acoustic impedance.

<Reference Comparative Example 2>

A uniform dispersion was obtained by mixing 70 mass parts of a furan resin (VF303 by Hitachi Chemical Co., Ltd.) as a curable resin, 20 mass parts of polymethyl methacrylate (PMMA) (particle size: 5 μm, thermal decomposition temperature: 400°C) as a dissipatable substance and 10 mass parts of graphite (flaky graphite, Nippon Graphite Co., mean particle size: 5 μm) as a carbonaceous powder, using a bead mill or Disper mixer.

To the obtained dispersion there was added 1 part by mass of para-toluenesulfonic acid as a curing agent, and the mixture was stirred to homogeneity and subjected to reduced-pressure defoaming treatment to obtain a precursor composition. The precursor composition was filled into a die with a diameter of 100 mm and a thickness of 30 mm, and cured. When the cured precursor composition was removed from the die and heat treated to a temperature of 1000°C under a nitrogen gas atmosphere, cracks were found to be present in the carbide, and a vitreous carbon molded body could not be obtained. Consequently it was not possible to measure the bending strength, flexural modulus and acoustic impedance.

<Reference Comparative Example 3>

A homogeneous solution was obtained by mixing 126 mass parts of a furan resin (VF303 by Hitachi Chemical Co., Ltd.) as a curable resin, and 20 mass parts of Solfit (boiling point: 174°C) and 30 mass parts of triethylene glycol (boiling point: 287°C) as solvents, and thoroughly stirring with a Disper mixer.

To the obtained solution there was added 10 mass parts of graphite (flaky graphite, Nippon Graphite Co., mean particle size: 5 μm) as a carbonaceous powder, and the mixture was uniformly dispersed using a bead mill or Disper mixer. To the obtained dispersion there was added 1 part by mass of para-toluenesulfonic acid as a curing agent, and the mixture was stirred to homogeneity and subjected to reduced-pressure defoaming treatment to obtain a precursor composition. The precursor composition was filled into a die with a diameter of 100 mm and a thickness of 30 mm, and cured. When the cured precursor composition was removed from the die and heat treated to a temperature of 1000°C under a nitrogen gas atmosphere, cracks were found to be present in the carbide, and a vitreous carbon molded body could not be obtained. Consequently it was not possible to measure the bending strength, flexural modulus and acoustic impedance.

The construction and evaluation results for each of the Reference Examples and Reference Comparative Examples are shown in Table 1. The evaluation under the "Solution state" column in Table 1 is "compatible" if the precursor composition before curing and before addition of the carbonaceous powder showed no undissolved matter when observed under an optical microscope at 100x or greater magnification, and otherwise "incompatible".

[Table 2]

Table 2

| | | Reference Example 1 | Reference Example 2 | Reference Example 3 | Reference Comp. Ex. 1 | Reference Comp. Ex. 2 | Reference Comp. Ex. 3 |
|---|---|---|---|---|---|---|---|
| Curable resin | Type | Furan resin | Furan resin | Furan resin | Furan resin | Furan resin | Furan resin |
| | Mass (mass parts) | 120 | 126 | 80 | 120 | 70 | 126 |
| Dissipatable substance | Type | PEG | PEG | PEG | - | PMMA | - |
| | Mass (mass parts ) | 14 | 10 | 4 | - | 20 | |
| Solvent | Type | BA | Solfit | BA | BA | - | Solfit |
| | | TEG | TrEG | DEG | TEG | - | TrEG |
| | Mass (mass parts) | 66 | 50 | 20 | 66 | - | 50 |
| Solution state | | Compatible | Compatible | Compatible | Compatible | Incompatible | Compatible |
| Carbonaceous powder | Type | | Graphite powder | Amorphous carbon powder | | Graphite powder | Graphite powder |
| | Mass (mass parts) | - | 14 | 80 | - | 10 | 10 |
| Curing agent | Type | PTS | PTS | PTS | PTS | PTS | PTS |
| | Mass (mass parts) | 2 | 1 | 3 | 1 | 1 | 1 |
| Thickness during die filling (mm) | | 25 | 30 | 30 | 30 | 30 | 30 |
| Thickness of carbon sheet (mm) | | 20 | 25 | 25 | Cracking | Cracking | Cracking |
| Acoustic impedance (MRay1) | | 4.5 | 4.4 | 5.3 | - | - | - |
| Bulk density (g/cm$^3$) | | 1.25 | 1.18 | 1.34 | - | - | - |
| Acoustic velocity (m/sec) | | 3636 | 3741 | 4018 | - | - | - |
| Pore diameter (nm) | | about 50 | about 50 | about 50 | - | - | - |
| Bending strength (MPa) | | 80 | 96 | 115 | - | - | - |

EP 4 270 991 A1

(continued)

| | Reference Example 1 | Reference Example 2 | Reference Example 3 | Reference Comp. Ex. 1 | Reference Comp. Ex. 2 | Reference Comp. Ex. 3 |
|---|---|---|---|---|---|---|
| Flexural modulus (GPa) | 19 | 17.5 | 24 | - | - | - |

In Reference Examples 1 to 3 which used precursor compositions in which the curable resin, dissipatable substance and solvent were compatible, it was possible to fabricate vitreous carbon molded bodies with thicknesses of 20 to 25 mm.

In contrast, in Reference Comparative Examples 1 and 3 which used precursor compositions without a dissipatable substance, and in Reference Comparative Example 2 which used a precursor composition without a solvent, it was not possible to fabricate a vitreous carbon molded body with a thickness of 20 mm or greater.

REFERENCE SIGNS LIST

10a, 10b Backing material matrix
12 Pores forming communicating pores
14 Vitreous carbon (particles)
100 Ultrasonic probe
102 Acoustic lens
104 Acoustic matching layer
106 Piezoelectric element
110 Backing material

**Claims**

1. A backing material for an ultrasonic probe which has:

    a carbonaceous matrix with communicating pores dispersed throughout the matrix, and
    a resin filling at least a portion of the communicating pores.

2. The backing material according to claim 1, wherein the acoustic impedance is 3.5 to 6.0 MRayl.

3. The backing material according to claim 1 or 2, wherein the thermal conductivity according to JIS R1611-**2010 is 7.0 W/m·K or greater**.

4. The backing material according to any one of claims 1 to 3, wherein the ultrasonic attenuation according to JIS Z 2354-2012 is -50 to -15 dB/cm.

5. The backing material according to any one of claims 1 to 4, wherein the matrix further comprises a carbonaceous powder.

6. The backing material according to any one of claims 1 to 5, wherein the density is 1.1 to 1.8 g/cm$^3$.

7. An ultrasonic probe equipped with an acoustic lens, an acoustic matching layer, a piezoelectric element and a backing material according to any one of claims 1 to 6, in that order.

8. A method for producing a backing material for an ultrasonic probe, wherein the method includes:

    providing a carbonaceous matrix with communicating pores dispersed throughout the matrix, and
    filling a resin into the communicating pores of the matrix.

9. The method according to claim 8, wherein provision of the matrix is by a method including the following steps:

    mixing a curable resin, a dissipatable substance and a solvent to compatibility, to prepare a precursor composition, and
    heat treating the precursor composition in a non-oxidizing atmosphere to carbonize the curable resin and form a matrix, and causing the dissipatable substance to disappear to form pores in the matrix.

# FIG. 1

(a)

(b)

# FIG. 2

## INTERNATIONAL SEARCH REPORT

| International application No. |
|---|
| **PCT/JP2021/045197** |

### A. CLASSIFICATION OF SUBJECT MATTER

*H04R 17/00*(2006.01)i; *H04R 31/00*(2006.01)i; *A61B 8/00*(2006.01)i
FI:   H04R17/00 330J; H04R31/00 330; A61B8/00

According to International Patent Classification (IPC) or to both national classification and IPC

### B. FIELDS SEARCHED

Minimum documentation searched (classification system followed by classification symbols)

H04R17/00; H04R31/00; A61B8/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2019-193151 A (MITSUBISHI PENCIL CO) 31 October 2019 (2019-10-31) paragraphs [0013]-[0057] | 1-2, 4-8 |
| A | paragraphs [0013]-[0057] | 3, 9 |
| Y | JP 2015-221214 A (HITACHI MEDICAL CORP) 10 December 2015 (2015-12-10) paragraphs [0063]-[0064] | 1-2, 4-8 |
| A | JP 2016-219662 A (KONICA MINOLTA INC) 22 December 2016 (2016-12-22) entire text, all drawings | 1-9 |
| A | WO 2011/148618 A1 (PANASONIC CORPORATION) 01 December 2011 (2011-12-01) entire text, all drawings | 1-9 |

☐ Further documents are listed in the continuation of Box C.     ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **08 February 2022** | **22 February 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

<div style="text-align: center">

**INTERNATIONAL SEARCH REPORT**
**Information on patent family members**

</div>

International application No.

**PCT/JP2021/045197**

| Patent document cited in search report | | | Publication date (day/month/year) | Patent family member(s) | | | Publication date (day/month/year) |
|---|---|---|---|---|---|---|---|
| JP | 2019-193151 | A | 31 October 2019 | EP | 3787314 | A1 | |
| | | | | paragraphs [0013]-[0057] | | | |
| | | | | WO | 2019/208118 | A1 | |
| | | | | CN | 111955019 | A | |
| | | | | KR | 10-2021-0005018 | A | |
| JP | 2015-221214 | A | 10 December 2015 | US | 2013/0285174 | A1 | |
| | | | | paragraphs [0075]-[0076] | | | |
| | | | | WO | 2012/093662 | A1 | |
| | | | | EP | 2662024 | A1 | |
| | | | | CN | 103298410 | A | |
| | | | | KR | 10-2013-0103590 | A | |
| JP | 2016-219662 | A | 22 December 2016 | (Family: none) | | | |
| WO | 2011/148618 | A1 | 01 December 2011 | US | 2012/0123274 | A1 | |
| | | | | entire text, all drawings | | | |
| | | | | EP | 2579615 | A1 | |
| | | | | CN | 102474692 | A | |

Form PCT/ISA/210 (patent family annex) (January 2015)

**EP 4 270 991 A1**

**Patent documents cited in the description**

- JP 2006033801 A
- JP 2013115537 A
- JP 2019193151 A
- JP S5964511 B